# DEMANDE DE BREVET EUROPEEN

(11) **EP 3 613 412 A1**
(43) Date de publication de la demande: **26.02.2020**
(21) Numéro de dépôt: 19201171.6
(22) Date de dépôt: 31.01.2011
(51) Int. Cl.: A61K 8/97, C11B 1/10, A61Q 19/00, A23L 33/105, A23L 33/115, A23L 33/10, A61K 8/92, C11C 1/02, C11C 3/00, A61K 8/9728, A61K 8/9722, A61K 8/9789, A61K 8/9794

(54) **EXTRACTION SOLIDE / LIQUIDE**

(30) Priorité: 29.01.2010 FR 1050646; 28.01.2011 FR 1150681
(62) Demande divisionnaire de: 11701288.0
(71) Demandeur: Minafin, 1435 Mont-Saint-Guibert (BE)
(72) Inventeur: SAUNOIS, Alex, 28210 NOGENT-LE-ROI (FR); LEGRAND, Jacques, 61290 NEUILLY SUR EURE (FR); MERCIER, Eglantine, 78120 RAMBOUILLET (FR)
(74) Mandataire: Plasseraud IP

(57) **Abrégé**

La présente invention concerne un procédé d'extraction solide / liquide d'une huile ou d'un beurre, notamment présentant une teneur importante en insaponifiable, contenue dans au moins une matière solide végétale ou un micro-organisme comprenant au moins les étapes suivantes :
- extraction solide / liquide d'au moins une matière solide végétale ou d'un micro-organisme par un premier système de solvants comprenant une teneur en solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (MeTHF), et leurs mélanges, d'au moins 50% en volume par rapport au volume total du système de solvants, et
- éventuellement récupération d'une fraction comprenant l'huile ou le beurre, notamment enrichi en insaponifiable,
une huile, un beurre ou une fraction insaponifiable obtenue par ledit procédé et des composition comprenant ladite huile ou fraction.

## Description

La présente invention concerne un procédé d'extraction solide / liquide d'une huile ou d'un beurre à partir d'une matière solide végétale ou d'un micro-organisme, en particulier ladite huile ou ledit beurre comprend une teneur importante en insaponifiable.

Les insaponifiables ou fractions insaponifiables d'un corps gras sont constitués de composés qui forment la partie d'un corps gras qui, après action prolongée d'une base alcaline, reste insoluble dans l'eau et peut être extraite par un solvant organique.

La plupart des insaponifiables d'huiles végétales comprennent des grandes familles de substances. Parmi ces grandes familles on peut citer les hydrocarbures saturés ou insaturés, les alcools aliphatiques ou terpéniques, les stérols, les tocophérols, les pigments caroténoïdes, xanthophiles, ainsi qu'une ou deux familles spécifiques dans le cas de certaines huiles.

Les procédés usuels d'obtention des insaponifiables des huiles végétales visent à extraire tout ou partie des grandes familles qui les composent, permettant de préparer les fractions partielles ou totales des insaponifiables.

Les fractions partielles ou totales d'insaponifiables sont notamment recherchées pour leurs propriétés pharmacologiques, cosmétiques et nutritionnelles.

Les procédés usuels d'obtention des insaponifiables des huiles végétales comprennent entre autre une étape de saponification de la matière grasse et une extraction du produit cible (l'insaponifiable) par un solvant organique.

Les solvants les plus communément utilisés pour extraire les huiles, en particulier riches en insaponifiable, de matières solides végétales sont les solvants aliphatiques, et en particulier l'hexane.

L'hexane présente notamment l'inconvénient d'être reprotoxique, il est notamment classé CMR de classe 3 dans la liste CMR UE1 ou dans la liste CMR UE2.

L'hexane présente encore l'inconvénient d'être dangereux en terme de manipulation, notamment du fait de ses propriétés physico-chimique, en particulier de son point éclair (-23,3°C) et/ou de sa température d'auto inflammation (233,9°C).

Enfin, les procédés impliquant ces solvants aliphatiques classiques, et notamment l'hexane peuvent être insatisfaisants en terme de rendement, par rapport à l'huile et/ou par rapport à la teneur de l'huile obtenue en insaponifiable, de sélectivité, de simplicité, de coût, de toxicité, de commodité, de nombre d'étapes, notamment d'extraction, et/ou de rapidité.

La présente invention vise donc à résoudre en tout ou partie les problèmes évoqués ci-dessus. En particulier, l'invention vise à fournir un procédé plus économique, plus direct, plus amical pour l'environnement, nécessitant une quantité de solvant organique plus faible, plus facile à mettre en œuvre, plus rapide, générant des conditions moins toxiques, permettant l'obtention d'huiles, notamment présentant une teneur importante en insaponifiable, avec un rendement et/ou une sélectivité au moins comparables, voire supérieurs, aux procédés déjà existants.

En particulier, il est souhaitable que le(s) solvant(s) impliqué(s) soi(en)t moins toxique(s), notamment non-classé(s) CMR, notamment CMR UE2, et/ou permette(nt) d'extraire les huiles avec un rendement et/ou une sélectivité au moins comparables aux rendements et sélectivités obtenus en utilisant les solvants aliphatiques classiques, et notamment l'hexane.

Les solvants dits « classés CMR » peuvent être ceux qui sont présentés dans la liste en annexes de la directive 2009/2/CE du 15 janvier 2009, notamment disponible à l'adresse http://eur-lex.europa.eu/LexUriServ/LexUriServ.do?uri=OJ:L:2009:011:0006:0082:FR:PDF, cette première liste étant appelée ci-après « liste CMR UE1 », ceux listés dans la Classification européenne réglementaire des produits chimiques cancérogènes, mutagènes et toxiques pour la reproduction - 31e ATP, 2009, notamment disponible à l'adresse http://www.prc.cnrs-gif.fr/en_telechargement/cmr31.pdf cette deuxième liste étant appelée ci-après « liste CMR UE2 », et/ou ceux listés dans la liste « Chemicals known or suspected to cause cancer or reproductive toxicity » du 1^{er} septembre 2009 établie par le « California department of public health, occupational health branch, California safe cosmetic program » liée à la « California Safe Cosmetics Act of 2005 » cette troisième liste étant appelée ci-après « liste CMR US ».

Lorsque dans le présent texte est utilisé l'expression liste CMR UE, on entend liste CMR UE1 et CMR UE2, et en particulier CMR UE2.

La présente invention a ainsi pour objet un procédé d'extraction solide / liquide d'une huile ou d'un beurre, notamment présentant une teneur importante en insaponifiable, contenue dans au moins une matière solide végétale ou d'un micro-organisme comprenant au moins les étapes suivantes :
- extraction solide / liquide d'au moins une matière solide végétale ou d'un micro-organisme par un premier système de solvants comprenant une teneur en solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (MeTHF), et leurs mélanges, d'au moins 50% en volume par rapport au volume total du système de solvant,
- éventuellement récupération d'une fraction comprenant l'huile ou le beurre, notamment enrichie en insaponifiable.

Les numéros CAS de ces différents solvants étant les suivants BTF : 98-08-8 ; BHF : 392-56-3 ; ETBE : 37-92-3 ; MTBE : 1634-04-4 ; HMDS : 107-46-0 ; TMS : 75-76-3 ; et MeTHF : 96-47-9.

Par « teneur importante en insaponifiable », on entend selon la présente invention que l'huile ou le beurre comprend au moins 1 % en masse, notamment au moins 2 % en masse, et en particulier au moins 3 % en masse des composés insaponifiables présents initialement dans la matière solide.

Le premier système de solvants peut comprendre une teneur de solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (MeTHF),et leurs mélanges, d'au moins 60 %, notamment au moins 75%, en particulier au moins 90%, plus particulièrement au moins 95%, encore plus particulièrement au moins 99% en volume par rapport au volume total du premier système de solvant.

En particulier, le premier système de solvants est constitué de solvant aromatique fluoré, notamment le trifluorotoluène (BTF) et l'hexafluorobenzène (BHF), de tert-butyl éther, notamment le 2-éthoxy-2-méthylpropane, encore appelé éthyl-tert-butyl-éther (ETBE) et le 2-méthoxy-2-méthylpropane ou méthyl-tert-butyléther (MTBE), de solvant comprenant au moins un atome de silicium, notamment l'hexaméthyldisiloxane (HMDS) et le tétraméthylsilane (TMS), de méthyl-tétrahydrofurane (MeTHF),ou d'un mélange de ceux-ci.

Le premier système de solvants peut comprendre une teneur en un solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (MeTHF),, et leurs mélanges, d'au moins 50 %, notamment au moins 75%, en particulier au moins 90%, plus particulièrement au moins 95%, encore plus particulièrement au moins 99% en volume par rapport au volume total du premier système de solvants.

Selon une variante, le premier système de solvants est constitué d'un solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS) et le méthyl-tétrahydrofurane (MeTHF).

L'invention concerne également un procédé d'obtention d'une fraction insaponifiable, notamment totale ou partielle, comprenant au moins les étapes suivantes :
- extraction solide / liquide d'au moins une matière solide végétale ou d'un micro-organisme par un premier système de solvants comprenant une teneur en solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (MeTHF),et leurs mélanges, d'au moins 50% en volume par rapport au volume total du système de solvants,
- éventuellement récupération d'une solution organique enrichie en huile ou en beurre, voire récupération de l'huile ou du beurre, notamment ladite huile ou ledit beurre est enrichi en insaponifiable,
- transformation de ladite huile ou dudit beurre en solution hydro-alcoolique, notamment via une étape choisie parmi les saponifications et les estérifications,
- extraction de la solution hydro-alcoolique dans laquelle la fraction grasse est séparée de la fraction insaponifiable par une extraction liquide / liquide ou par une distillation, et
- récupération de la fraction insaponifiable, notamment partielle ou totale.

Par « fraction totale », on entend au sens de la présente invention le fait que cette fraction comprend toutes les familles de substances composant l'insaponifiable présentes dans l'huile ou dans le beurre végétal ou dans le micro-organisme considéré.

Par « fraction partielle », on entend au sens de la présente invention le fait que cette fraction comprend au moins une des familles de substances composant l'insaponifiable présente dans l'huile ou dans le beurre végétal ou dans le un micro-organisme considéré.

Le premier système de solvants est tel que défini dans le cas du procédé d'extraction solide / liquide de l'huile ou du beurre.

La transformation de ladite huile ou dudit beurre en solution hydro-alcoolique peut être effectuée dans un système de solvants classique.

Selon une variante particulière, la transformation de ladite huile ou dudit beurre en solution hydro-alcoolique peut être effectuée dans un deuxième système de solvants comprenant, voire consistant en, au moins un solvant du premier système de solvants, en particulier le méthyltétrahydrofurane (MeTHF).

Plus particulièrement, la transformation peut être effectuée sans purification totale de l'huile ou du beurre. En particulier, la transformation est effectuée directement sur la base de la solution organique enrichie en huile ou en beurre, notamment comprenant au moins 2% en masse, en particulier au moins 5 % en masse, voire au moins 10% en masse d'huile par rapport à la masse totale de la solution organique enrichie en huile ou en beurre.

Selon une première variante, la transformation est réalisée sur une fraction, notamment en partie évaporée, à laquelle est ajoutée moins de 50 % en masse d'autres solvants, voire il n'est pas ajouté d'autres solvants.

Selon une autre variante au moins 10 % d'au moins un autre solvant, comme des alcools en C2 à C4, notamment l'éthanol, le n-propanol, l'iso-propanol, le butanol, en particulier le n-butanol, le méthyltétrahydrofurane (MeTHF) et leurs mélanges, peuvent être ajoutés solution organique enrichie en huile, notamment évaporée.

Avantageusement, le premier et le deuxième système de solvants comprend du méthyltétrahydrofurane (MeTHF). Dans ce cas le procédé peut avoir l'avantage d'être amélioré par rapport aux procédés classiques, par exemple en termes de rendements, de toxicité (solvant non CMR), et de sélectivité.

Dans le cas où l'extraction de la solution hydro-alcoolique est effectuée par une extraction liquide / liquide, celle-ci peut être faite avec un troisième système de solvants défini de la même manière que le premier système de solvants, et en particulier avec en partie les mêmes solvants que ceux utilisés dans le premier et/ou le deuxième système de solvants.

Dans ce dernier cas, le procédé est avantageux en termes économiques et/ou de temps dans la mesure où cela implique notamment une gestion plus aisée de l'approvisionnement en solvants et/ou en contrôle qualité de ceux-ci.

De manière générale, le procédé peut être plus économique, plus direct, plus amical pour l'environnement nécessitant une quantité de solvant organique plus faible, plus facile à mettre en œuvre, plus rapide, générant des conditions moins toxiques, permettant l'obtention d'huiles, notamment présentant une teneur importante en insaponifiable, avec un rendement et/ou une sélectivité au moins comparables, voire supérieurs, aux procédés déjà existants.

La partie saponification et extraction de l'insaponifiable peut notamment être effectuée selon les procédures décrites dans FR 1 246 633.

La matière solide végétale ou le micro-organisme utilisée dans les présents procédés peut provenir du soja, du colza, du maïs, du tournesol, du sésame, du lupin, du coton, de la noix de coco, d'olive, d'avocat, de cacao, d'illipé, de karité, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette, de macro-algues, de micro-algues, telles que les Haematocococcus, Dunaliella, Spirulina, Chorella, et/ou de micro-organismes, notamment marin, d'eau douce ou terrestre, en particulier de levures, de moisissures, et plus particulièrement, de bactéries, et leurs mélanges.

Typiquement, les teneurs de fraction insaponifiable obtenues s'échelonnent de 2 à 10% dans l'huile d'avocat, sont d'environ 0,5% dans l'huile de coco, d'environ 1% dans l'huile de soja et d'environ 1 % dans l'huile d'olive.

L'Homme de l'Art connaît les procédés à mettre en œuvre pour extraire la fraction insaponifiable d'une huile ou d'un beurre végétal ou d'un micro-organisme et sait les appliquer à la partie transformation, extraction et/ou récupération de l'insaponifiable de la présente invention.

Parmi l'art antérieur portant sur cette partie, on peut citer en particulier le procédé de préparation d'insaponifiable d'huile d'avocat tel que décrit et revendiqué dans le brevet FR 2 678 632 au nom des Laboratoires Pharmascience. Ce procédé permet d'obtenir un insaponifiable d'huile d'avocat riche en fraction furanique, encore appelée fraction H, en comparaison aux procédés classiques de préparation d'insaponifiable d'huile d'avocat.

Ainsi, l'insaponifiable d'huile d'avocat peut être préparé à partir du fruit préalablement traité thermiquement, avant l'extraction de l'huile et la saponification, comme décrit dans le brevet FR 2 678 632.

Ce traitement thermique consiste en un séchage contrôlé du fruit, frais de préférence, pendant au moins quatre heures, avantageusement au moins 10 heures, de préférence entre environ 24 et environ 48 heures, à une température de préférence d'au moins environ 80°C et de préférence comprise entre environ 80 et environ 120°C.

On peut également citer le procédé de préparation d'insaponifiable d'huile de soja, obtenu à partir d'un concentrât d'insaponifiable d'huile de soja.

Ledit concentrât d'insaponifiable peut être préparé par distillation moléculaire selon un procédé tel que décrit pour l'huile de lupin dans la demande de brevet FR 2 762 512, mais adapté à l'huile de soja.

Dans ce procédé, l'huile de soja est distillée dans un distillateur moléculaire de type centrifuge ou à film raclé, à une température comprise entre environ 210 et 250°C et sous un vide poussé, compris entre 0,01 et 0,001 millimètres de mercure (soit 0,13 à 1,3 Pa).

Le distillat obtenu présente une teneur en insaponifiable comprise entre 5 et 40% en masse et constitue donc un concentrât d'insaponifiable d'huile de soja.

Le concentrât est ensuite saponifié par une base telle que la potasse ou la soude en milieu polaire, notamment alcoolique, de préférence de l'éthanol, du n-propanol, de l'isopropanol, du butanol, en particulier du n-butanol, du méthyl-tétrahydrofurane (MeTHF), ou un mélange de ceux-ci, puis il est soumis à une ou plusieurs extractions par le premier système de solvants.

La solution d'extraction obtenue est de préférence ensuite centrifugée, filtrée puis lavée à l'eau pour éliminer les traces résiduelles d'alcalinité.

Le solvant d'extraction est évaporé soigneusement pour récupérer l'insaponifiable. Enfin, avant sa saponification, l'huile ou le beurre peut être préalablement enrichie en insaponifiable en séparant une majorité des constituants de l'insaponifiable que l'on récupère dans un concentrât. Différentes méthodes peuvent être utilisées : cristallisation par le froid, extraction liquide / liquide, ou encore distillation moléculaire.

La concentration préalable de l'huile ou du beurre en insaponifiable permet de diminuer les volumes d'huile ou de beurre à saponifier.

La distillation moléculaire est particulièrement préférée, étant réalisée de préférence à une température comprise entre environ 180 et environ 230°C en maintenant une pression comprise entre 10⁻³ et 10⁻² mm Hg et de préférence de l'ordre de 10⁻³ mm Hg.

La concentration en insaponifiable du distillat peut atteindre 60% en masse par rapport à la masse totale.

Tout particulièrement, la présente invention concerne un procédé tel que décrit dans la présente description dans lequel l'insaponifiable obtenu est choisi parmi un insaponifiable de soja, un insaponifiable d'avocat, notamment un insaponifiable d'avocat enrichi en fraction furanique et/ou un insaponifiable d'avocat enrichi en fraction stérolique, et plus particulièrement un mélange d'insaponifiables d'avocat et de soja (ASU).

Par « insaponifiable enrichi en fraction X », on entend au sens de la présente invention que la teneur en fraction X dans l'insaponifiable est augmentée, en particulier d'au moins 10 % en masse, notamment d'au moins 50 % en masse, tout particulièrement d'au moins 80 % en masse.

La présente invention a encore pour objet une huile ou un beurre dépourvu de solvants classés dans la liste CMR UE1, liste CMR UE2 et/ou US, en particulier ladite huile ou ledit beurre est obtenu par le procédé selon la présente invention.

La présente invention a encore pour objet une fraction insaponifiable, notamment partielle ou totale, dépourvue de solvants classés dans la liste CMR UE1, UE2 et/ou US, en particulier ladite fraction est obtenue par le procédé d'extraction selon la présente invention.

La présente invention concerne encore l'utilisation de cette fraction, de ce beurre ou de cette huile pour la préparation d'une composition, notamment pharmaceutique, alimentaire et/ou cosmétique, ou encore d'un complément alimentaire.

La présente invention a encore pour objet une composition, notamment alimentaire, cosmétique ou pharmaceutique, ou encore un complément alimentaire, comprenant au moins une huile, un beurre ou une fraction insaponifiable d'au moins une huile ou d'un beurre végétal ou d'un micro-organisme, ladite huile, beurre ou fraction étant dépourvue de solvants classés dans la liste CMR UE1, UE2 et/ou US et/ou ladite huile, beurre ou fraction est susceptible d'être obtenue, ou directement obtenue, par le procédé selon l'invention, et ladite composition comprenant éventuellement un excipient, en particulier cosmétiquement, alimentairement ou pharmaceutiquement acceptable.

Selon un mode de réalisation particulier, la présente invention concerne une composition, notamment pharmaceutique, alimentaire ou cosmétique, ou encore un complément alimentaire, comprenant au moins un insaponifiable, en particulier un insaponifiable de soja, un insaponifiable d'avocat, tout particulièrement un insaponifiable d'avocat riche en fraction furanique et/ou un insaponifiable d'avocat riche en fraction stérolique, et encore plus particulièrement un mélange d'insaponifiables d'avocat et de soja (ASU) susceptible d'être obtenu ou directement obtenu par le procédé selon l'invention.

Les compositions pharmaceutiques peuvent être destinées à la prévention et/ou au traitement de troubles du tissu conjonctif, notamment de l'arthrose, des parodontopathies et/ou du vieillissement cutané.

Les compositions alimentaires, ou compléments alimentaires peuvent être destinés à la prévention et/ou au traitement de troubles du tissu conjonctif, notamment de l'arthrose, des parodontopathies, du vieillissement cutané et/ou et inflammation cutanée.

Les compositions cosmétiques peuvent être destinées à la prévention et/ou au traitement des troubles cutanées de l'épiderme, du derme et/ou de l'hypoderme.

Par « dépourvu de solvants classés dans la liste CMR UE1, UE2 et/ou US », on entend au sens de la présente invention une teneur totale en solvants classés dans la liste CMR UE1, UE2 et/ou US inférieure à 10 ppm, notamment inférieure à 5 ppm, en particulier inférieure à 2 ppm, voire inférieure à 1 ppm.

La présente invention vise encore un procédé de traitement cosmétique tel que l'on applique de façon topique la composition cosmétique selon l'invention et aussi l'utilisation d'une huile, d'un beurre, ou d'insaponifiable d'une huile ou d'un beurre végétal ou d'un micro-organisme obtenu selon la présente invention pour la fabrication d'un médicament, en particulier destiné à traiter ou à prévenir des troubles du tissu conjonctif, et notamment l'arthrose.

Bien entendu, les différentes caractéristiques exposées dans la présente description peuvent être combinées entre elles.

A titre d'exemples illustrant la présente invention, les expérimentations suivantes ont été effectuées.

### EXEMPLES

Dans tous les exemples, un essai de référence utilisant l'hexane a été réalisé.

### Exemple 1 : extraction à partir d'avocats déshydratés

Une extraction d'avocats déshydratés a été effectuée avec de l'hexane (référence) et avec les solvants suivants : HMDS, MeTHF, BTF, BHF, MTBE, ETBE.

De l'avocat déshydraté est broyé et introduit dans une cartouche de cellulose (30 à 40 g). L'extraction est réalisée dans un appareil de type Soxhlet (BUCHI B-811). Quatre extractions sont alors lancées en parallèle et correspondent chacune à 20 cycles d'extraction / siphonage. Une fois l'extraction finalisée, le solvant d'extraction est évaporé et le résidu désolvanté est pesé. Les rendements massiques sont ensuite comparés. Les résultats sont présentés dans le tableau 1.

**Tableau 1**

| **Solvant d'extraction** | **Rendement en huile (% m/m)** | **Teneur en insaponifiable de l'huile (% m/m)** |
|---|---|---|
| Hexane | 61,1 | 1,88 |
| ETBE | 59,7 | 2,75 |
| MeTHF | 61,3 | 3,05 |
| HFB | 59,3 | 2,32 |
| BTF | 64,0 | 2,41 |
| HMDS | 60,5 | 1,92 |
| MTBE | 65,3 | 1,92 |

Ceci montre que les solvants selon l'invention présentent une capacité extractive équivalente à celle de l'hexane.

La teneur en insaponifiable de l'huile extraite est plus élevée dans le cas des solvants selon l'invention que dans le cas de l'hexane; celle-ci est augmentée de 50% dans le cas de l'ETBE et du MeTHF.

### Exemple 2 : extraction à partir de poudre de pulpe d'avocats lyophilisée

Une extraction de poudre de pulpe d'avocats lyophilisée a été effectuée selon le mode opératoire décrit dans l'exemple 1 avec de l'hexane (référence) et avec les solvants suivants : HMDS, MeTHF, BTF, MTBE, ETBE. Les résultats sont présentés dans le tableau 2.

**Tableau 2**

| **Solvant d'extraction** | **Rendement en huile (% m/m)** | **Teneur en insaponifiable de l'huile (% m/m)** |
|---|---|---|
| Hexane | 70,1 | 2,15 |
| ETBE | 69,6 | 2,25 |
| MeTHF | 71,7 | 3,02 |
| BTF | 67,3 | 2,22 |
| HMDS | 68,8 | 2,22 |
| MTBE | 70,1 | 2,35 |

Ceci montre que les solvants selon l'invention présentent une capacité extractive équivalente à celle de l'hexane.

La teneur en insaponifiable de l'huile extraite est plus élevée dans le cas des solvants selon l'invention que dans le cas de l'hexane ; celle-ci est augmentée de 50% dans le cas du MeTHF.

### Exemple 3 : extraction d'huile à partir d'avocats déshydratés

Une extraction d'avocats déshydratés est effectuée avec de l'hexane (référence) et les solvants suivants : BTF et ETBE.

De l'avocat déshydraté est broyé et introduit dans un cartouche de cellulose (30 à 40 g). L'extraction est réalisée dans un appareil de type Soxhlet (BUCHI B-811). Quatre extractions sont alors lancées en parallèle, la première correspondant à 5 cycles d'extraction, la seconde, à 10 cycles, la troisième à 15 cycles et la quatrième à 20 cycles. Une fois l'extraction finalisée, le solvant d'extraction est évaporé et le résidu désolvanté est pesé. Les rendements massiques sont ensuite comparés.

Les résultats sont présentés dans le tableau 3 suivant.

**Tableau 3**

| Solvant | Hexane | | | | ETBE | | | | BTF |
|---|---|---|---|---|---|---|---|---|---|
| Cycles | 5 | 10 | 15 | 20 | 5 | 10 | 15 | 20 | 5 |
| **Rendement en huile (% m/m)** | 18,3% | 31,3% | 33,1% | 40,9% | 17,6% | 26,8% | 30,2% | 40,3% | 39,0% |

Les rendements massiques obtenus avec l'ETBE sont similaires à ceux obtenus avec la référence hexane : l'ETBE se présente donc comme une alternative à l'utilisation de l'hexane dans des procédés d'extraction solide / liquide.

Le pouvoir extractif du BTF permet d'obtenir en seulement 5 cycles d'extraction le même rendement que ceux obtenus en 20 cycles d'extraction avec l'hexane ou l'ETBE. Le BTF se présente donc comme une bonne alternative à l'utilisation de l'hexane en permettant de diminuer de façon conséquente la quantité de solvant et/ou les temps de mise en contact à mettre en œuvre.

### Exemple 4 : extraction à partir de Chorella déshydratée

Une extraction de poudre de Chorella déshydratée a été effectuée selon le mode opératoire décrit dans l'exemple 1 avec de l'hexane (référence) et avec les solvants suivants : BTF et MTBE. Les résultats sont présentés dans le tableau 4.

**Tableau 4**

| **Solvant d'extraction** | **Rendement en huile (% m/m)** | **Teneur en insaponifiable de l'huile (% m/m)** |
|---|---|---|
| Hexane | 70 | 3,0 |
| MTBE | 65,4 | 3,7 |
| BTF | 72 | 3,4 |

Ceci montre que les solvants selon l'invention présentent une capacité extractive équivalente à celle de l'hexane.

La teneur en insaponifiable de l'huile extraite est plus élevée dans le cas des solvants selon l'invention que dans le cas de l'hexane.

### OBJET

Objet 1. Procédé d'extraction solide / liquide d'une huile ou d'un beurre, notamment présentant une teneur importante en insaponifiable, contenue dans au moins une matière solide végétale ou un micro-organisme comprenant au moins les étapes suivantes :
   - Extraction solide / liquide d'au moins une matière solide végétale ou d'un micro-organisme par un premier système de solvants comprenant une teneur en solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (methf), et leurs mélanges, d'au moins 50% en volume par rapport au volume total du système de solvants,
   - Eventuellement récupération d'une fraction comprenant l'huile ou le beurre.
Objet 2. Procédé d'obtention d'une fraction insaponifiable, notamment totale ou partielle, comprenant au moins les étapes suivantes :
   - Extraction solide / liquide d'au moins une matière solide végétale ou d'un micro-organisme par un premier système de solvants comprenant une teneur en solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (methf),et leurs mélanges, d'au moins 50% en volume par rapport au volume total du système de solvants,
   - Eventuellement récupération d'une solution organique enrichie en huile ou en beurre, voire récupération de l'huile ou du beurre,
   - Transformation de ladite huile ou dudit beurre en solution hydro-alcoolique, notamment via une étape choisie parmi les saponifications et les estérifications,
   - Extraction de la solution hydro-alcoolique dans laquelle la fraction grasse est séparée de la fraction insaponifiable par une extraction liquide / liquide et
   - Récupération de la fraction insaponifiable.
Objet 3. Procédé selon l'objet 1 ou 2, caractérisé en ce que le premier système de solvants comprend une teneur de solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (methf),et leurs mélanges, d'au moins 60 %, notamment au moins 75%, en particulier au moins 90%, plus particulièrement au moins 95%, encore plus particulièrement au moins 99% en volume par rapport au volume total du premier système de solvants.
Objet 4. Procédé selon l'un quelconque des objets 1 à 3, caractérisé en ce que le premier système de solvants est constitué de 2-méthoxy-2-méthylpropane (MTBE), de 2-éthoxy-2-méthylpropane (ETBE), de méthyl-tétrahydrofurane (methf), d'hexaméthyldisiloxane (HMDS), de trifluorotoluène (BTF), d'hexafluorobenzène (BHF), de tétraméthylsilane (TMS) ou d'un mélange de ceux-ci.
Objet 5. Procédé selon l'un quelconque des objets 1 à 4, caractérisé en ce que le premier système de solvants comprend une teneur en un solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (methf), et leurs mélanges, d'au moins 50 %, notamment au moins 75%, en particulier au moins 90%, plus particulièrement au moins 95%, encore plus particulièrement au moins 99% en volume par rapport au volume total du premier système de solvants.
Objet 6. Procédé selon l'un quelconque des objets 2 à 5, caractérisé en ce que la transformation de ladite huile en solution hydro-alcoolique est effectuée dans un deuxième système de solvants comprenant, voire consistant en, au moins un solvant du premier système de solvants, en particulier le méthyl-tétrahydrofurane (methf).
Objet 7. Procédé selon l'un quelconque des objets 2 à 6, caractérisé en ce que la transformation est effectuée directement sur la base de la solution organique enrichie en huile ou en beurre, notamment comprenant au moins 2% en masse, en particulier au moins 5 % en masse, voire au moins 10% en masse d'huile par rapport à la masse totale de la solution organique enrichie en huile ou en beurre.
Objet 8. Procédé selon l'objet 7, caractérisé en ce que la transformation est réalisée sur une fraction, notamment en partie évaporée, à laquelle il est ajoutée moins de 50 % en masse d'autres solvants, voire il n'est pas ajouté d'autres solvants.
Objet 9. Procédé selon l'objet 7, caractérisé en ce qu'au moins 10 % d'au moins un autre solvant, comme des alcools en C2 à C4, notamment l'éthanol, l'iso-propanol, le butanol, en particulier le n-butanol, le méthyl-tétrahydrofurane (methf) et leurs mélanges, peuvent être ajoutés à la fraction enrichie en huile, notamment évaporée.
Objet 10. Procédé selon l'un quelconque des objets 6 à 9, caractérisé en ce que le premier et le deuxième système de solvants comprennent du methf.
Objet 11. Procédé selon l'un quelconque des objets 1 à 10, caractérisé en ce que l'extraction de la solution hydro-alcoolique est effectuée par une extraction liquide / liquide faite avec un troisième système de solvants comprenant en tout ou partie les mêmes solvants que ceux utilisés dans le premier et/ou le deuxième système de solvants.
Objet 12. Procédé selon l'un quelconque des objets 1 à 11, caractérisé en ce que la matière solide végétale ou le micro-organisme provient du soja, du colza, du maïs, du tournesol, du sésame, du lupin, du coton, de la noix de coco, d'olive, d'avocat, de cacao, d'illipé, de karité, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette, de macro-algues, de micro-algues, telles que les Haematocococcus, Dunaliella, Spirulina, Chorella, et/ou de micro-organismes, notamment marin, d'eau douce ou terrestre, en particulier de levures, de moisissures, et plus particulièrement, de bactéries, et leurs mélanges.
Objet 13. Procédé selon l'un quelconque des objets 1 à 12, caractérisé en ce l'insaponifiable obtenu est choisi parmi un insaponifiable de soja, un insaponifiable d'avocat, notamment un insaponifiable d'avocat enrichi en fraction furanique et/ou un insaponifiable d'avocat enrichi en fraction stérolique, et plus particulièrement un mélange d'insaponifiables d'avocat et de soja (ASU).
Objet 14. Huile ou fraction insaponifiable dépourvue de solvants classés dans la liste CMR UE1, UE2 et/ou US, ladite fraction ou ladite huile étant susceptible d'être obtenue selon l'un quelconque des objets 1 à 13, en particulier ladite huile ou fraction est obtenue par un procédé d'extraction selon l'un quelconque des objets 1 à 13.
Objet 15. Composition, notamment alimentaire, cosmétique ou pharmaceutique, ou complément alimentaire, comprenant au moins une huile, un beurre ou une fraction insaponifiable d'au moins une huile ou d'un beurre végétal ou d'un micro-organisme, ladite fraction, ledit beurre ou ladite huile étant susceptible d'être obtenue selon l'un quelconque des objets 1 à 13, ladite huile, ledit beurre ou ladite fraction étant dépourvue de solvants classés dans la liste CMR UE1, UE2 et/ou US, et ladite composition comprenant éventuellement un excipient, en particulier cosmétiquement, alimentairement ou pharmaceutiquement acceptable.
Objet 16. Composition, notamment pharmaceutique alimentaire ou cosmétique, ou encore un complément alimentaire, comprenant au moins un insaponifiable, en particulier un insaponifiable de soja, un insaponifiable d'avocat, tout particulièrement un insaponifiable d'avocat riche en fraction furanique et/ou un insaponifiable d'avocat riche en fraction stérolique, et encore plus particulièrement un mélange d'insaponifiables d'avocat et de soja (ASU) susceptible d'être obtenu, ou directement obtenu, par le procédé selon l'un quelconque des objets 1 à 13.
Objet 17. Composition pharmaceutique telle que définie selon l'objet 15 ou 16 caractérisée en ce qu'elle est destinée à la prévention et/ou au traitement de troubles du tissu conjonctif, notamment de l'arthrose, des parodontopathies et/ou du vieillissement cutané.
Objet 18. Composition alimentaire, ou complément alimentaire, tel que défini selon l'objet 15 ou 16 caractérisée en ce qu'il est destiné à la prévention et/ou au traitement de troubles du tissu conjonctif, notamment de l'arthrose, des parodontopathies, du vieillissement cutané et/ou et inflammation cutanée.
Objet 19. Composition cosmétique telle que définie selon l'objet 15 ou 16 caractérisée en ce qu'elle est destinée à la prévention et/ou au traitement des troubles cutanées de l'épiderme, du derme et/ou de l'hypoderme.

## Revendications

1. Huile issue d'une matière solide végétale ou d'un micro-organisme d'une comprenant au moins 1 % en masse de composés insaponifiables présents initialement dans ladite matière solide végétale ou ledit micro-organisme.

2. Huile selon la revendication 1 dépourvue de solvants classés dans la liste CMR UE1, UE2 et/ou US.

3. Huile susceptible d'être obtenue selon un procédé d'extraction solide / liquide comprenant au moins les étapes suivantes :
- extraction solide / liquide d'au moins une matière solide végétale ou d'un micro-organisme par un premier système de solvants comprenant une teneur en solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (MeTHF), et leurs mélanges, d'au moins 50% en volume par rapport au volume total du système de solvants, et
- récupération d'une fraction comprenant l'huile.

4. Huile selon la revendication 3 dans laquelle le premier système de solvant comprend une teneur en méthyl-tétrahydrofurane (MeTHF), d'au moins 50% en volume par rapport au volume total du système de solvants.

5. Huile selon l'une quelconque des revendications 1 à 4, dans laquelle la matière solide végétale ou le micro-organisme provient du soja, du colza, du maïs, du tournesol, du sésame, du lupin, du coton, de la noix de coco, d'olive, d'avocat, de cacao, d'illipé, de karité, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette, de macro-algues, de micro-algues, telles que les Haematococcus, Dunaliella, Spirulina, Chorella, et/ou de micro-organismes, notamment marin, d'eau douce ou terrestre, en particulier de levures, de moisissures, et plus particulièrement, de bactéries, et leurs mélanges.

6. Composition comprenant une huile telle que définie selon l'une quelconque des revendications 1 à 5.

7. Matière solide déshuilée susceptible d'être obtenue selon un procédé d'extraction solide / liquide comprenant au moins les étapes suivantes :
- extraction solide / liquide d'au moins une matière solide végétale ou d'un micro-organisme par un premier système de solvants comprenant une teneur en solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (MeTHF), et leurs mélanges, d'au moins 50% en volume par rapport au volume total du système de solvants,
- récupération de la matière solide dégraissée.

8. Matière solide déshuilée selon la revendication 7 dans laquelle la matière solide végétale provient du soja, du colza, du maïs, du tournesol, du sésame, du lupin, du coton, de la noix de coco, d'olive, d'avocat, de cacao, d'illipé, de karité, de palmiste, d'arachide, de coprah, de lin, de ricin, de pépins de raisin, de pépins de courge, de pépins de cassis, de pépins de melon, de pépins de tomate, de pépins de citrouille, d'amande, de noisette, de noix, d'onagre, de bourrache, de carthame, de cameline, d'oeillette.

9. Matière solide déshuilée selon la revendication 7 ou 8 dans laquelle le premier système de solvant comprend une teneur en méthyl-tétrahydrofurane (MeTHF), d'au moins 50% en volume par rapport au volume total du système de solvants.

10. Micro-organisme déshuilé susceptible d'être obtenu selon un procédé d'extraction solide / liquide comprenant au moins les étapes suivantes :
- extraction solide / liquide d'au moins un micro-organisme par un premier système de solvants comprenant une teneur en solvant choisi parmi les solvants aromatiques fluorés, notamment le trifluorotoluène (BTF) et l'hexafluorobenzène (BHF), les tert-butyl éthers, notamment le 2-éthoxy-2-méthylpropane, encore appelé éthyl-tert-butyl-éther (ETBE) et le 2-méthoxy-2-méthylpropane ou méthyl-tert-butyléther (MTBE), les solvants comprenant au moins un atome de silicium, notamment l'hexaméthyldisiloxane (HMDS) et le tétraméthylsilane (TMS), le méthyl-tétrahydrofurane (MeTHF), et leurs mélanges, d'au moins 50% en volume par rapport au volume total du système de solvants,
- récupération de la matière solide dégraissée.

11. Micro-organisme déshuilé selon la revendication 10 dans lequel le micro-organisme provient de macro-algues, de micro-algues, telles que les Haematococcus, Dunaliella, Spirulina, Chorella, et/ou de micro-organismes, notamment marin, d'eau douce ou terrestre, en particulier de levures, de moisissures, et plus particulièrement, de bactéries, et leurs mélanges.

12. Micro-organisme selon la revendication 10 ou 11 dans lequel le premier système de solvant comprend une teneur en méthyl-tétrahydrofurane (MeTHF), d'au moins 50% en volume par rapport au volume total du système de solvants.
